# FASCICULE DE BREVET EUROPEEN

(11) **EP 1 293 215 B1**
(45) Date de publication et mention de la délivrance du brevet: **11.04.2007**
(21) Numéro de dépôt: 02291725.6
(22) Date de dépôt: 09.07.2002
(51) Int. Cl.: A61L 2/18, A61L 12/12, C07C 409/00

(54) **Procédé de préparation d'une composition désinfectante, procédé de désinfection de dispositifs médicaux**
Verfahren zur Herstellung einer desinfizierenden Zusammensetzung, Desinfektionsverfahren für medizinische Vorrichtungen
Process for the production of a disinfecting composition, process for disinfection of medical devices

(30) Priorité: 12.09.2001 FR 0111796
(43) Date de publication de la demande: 19.03.2003
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Le Rouzic, Daniel, 95120 Ermont (FR); Gamet, Jean-Claude, 71460 Saint Martin du Tartre (FR)
(74) Mandataire: Conan, Philippe Claude

(56) Documents cités:
- EP-A- 0 395 296
- WO-A-00/30690
- WO-A-01/10215
- US-B1- 6 171 551

## Description

L'invention a pour objet un nouveau procédé de désinfection de dispositifs médicaux et en particulier de désinfection des endoscopes mettant en oeuvre ladite solution, qu'il s'agisse d'un procédé manuel ou d'un procédé en machines et notamment, en machines à désinfecter ou à laver et à désinfecter des endoscopes.

L'évolution actuelle des soins médicaux conduit à la multiplication des techniques exploratrices mettant en oeuvre des dispositifs de plus en plus petits, souvent fabriqués avec des matériaux thermosensibles. Il s'ensuit que ces dispositifs ne peuvent pas être stérilisés à chaud et que, lorsqu'ils ne sont pas à usage unique, ils doivent être nettoyés et désinfectés chimiquement après chaque utilisation. Selon la nature du tissu avec lequel le dispositif médical est en contact lors de son utilisation, on établit trois niveaux de risques infectieux, auxquels correspondent des niveaux de traitement de désinfection requis pour un spectre d'activité à atteindre. Au haut niveau de risque infectieux, correspond un traitement de haut niveau qui doit être efficace contre tous les types microorganismes, y compris les spores bactériennes. A titre d'exemples de matériels qui doivent subir ce traitement de haut niveau, à défaut de pouvoir subir une stérilisation à chaud, on peut citer les instrumentations et moteurs de microchirurgie et coelio - chirurgie, les pièces à mains en dentisterie, en stomatologie et en chirurgie, le matériel biofeedback en urologie, les dispositifs médicaux en ophtalmologie et de nombreux endoscopes.

Pour respecter en France, la circulaire de la Direction générale de la santé DGS/5C/DHOS/E2/2001/138 du 14 mars 2001, relative aux précautions à observer lors de soins en vue de réduire les risques de transmission d'agents transmissibles non conventionnels (ATNC), le traitement des dispositifs médicaux recyclables non autoclavables doit comprendre au moins une étape de nettoyage soigneux, une étape d'inactivation des ATNC et une étape de stérilisation ou de désinfection destinée à détruire les agents infectieux conventionnels et qui peut être réalisée dans le même temps que l'étape d'inactivation des ATNC, lorsque le procédé le permet.

Généralement, le traitement du dispositif comprend l'ensemble des étapes suivantes :
(a) un pré-traitement, dont l'objectif est de faciliter les étapes ultérieures en empêchant, par exemple, les salissures de sécher. Ainsi dès que leur utilisation est terminée, les canaux des instruments médicaux en comportant, sont au plus tôt irrigués et/ou immergés dans une solution détergente éventuellement bactéricide ;
(b) un nettoyage en une ou deux étapes successives, dont l'objectif est d'éliminer les salissures. Il va conjuguer l'action physico-chimique de la solution utilisée et les actions mécaniques effectuées manuellement ou par des machines ;
(c) la désinfection chimique, impliquant la mise en contact du dispositif à désinfecter avec une solution désinfectante efficace contre les agents infectieux conventionnels et contre les ATNC ;
(d) le rinçage final, dont l'objectif est d'éliminer tout résidu de produit, tout en évitant les re-contaminations ; ceci implique l'utilisation d'une eau adaptée à cette exigence qui, selon le niveau de risque, sera stérile ou filtrée ; et, lorsque le dispositif n'est pas immédiatement réutilisé,
(e) le séchage, puis
(f) le stockage, ces deux dernières étapes devant, elles aussi, être effectuées dans des conditions évitant les re-contaminations du dispositif.

L'étape (c) peut être faite, soit par un procédé manuel, soit par un procédé semi-automatique ou automatique.

Un procédé manuel consiste, soit en l'immersion dans une solution désinfectante lorsque le dispositif est submersible, soit en l'application d'un désinfectant à l'aide d'un support préalablement humidifié avec une solution désinfectante lorsque le dispositif n'est pas submersible, soit en la pulvérisation dirigée sur le dispositif, d'une solution désinfectante. Un procédé semi-automatique consiste en une pulvérisation au moyen d'un pulvérisateur électrique, permettant une désinfection de contact, par "dispersât dirigé" d'une solution désinfectante. Ce procédé est souvent utilisé pour nettoyer les surfaces.

Un procédé automatique met en oeuvre, soit des appareils spécifiques à un instrument donné, comme les machines à laver les endoscopes, soit des aérolyseurs pour la désinfection par voie aérienne des surfaces de locaux et des équipements qu'ils referment.

Dans la désinfection automatique des endoscopes, les étapes (b) à (e) sont réalisées dans des machines spécifiques à ces instruments. De tels appareils sont censés être plus fiables, tant en termes de traçabilité que de reproductibilité du traitement ; certains d'entre eux ne réalisent que l'étape de désinfection, mais la plupart effectue à la fois le nettoyage, la désinfection, le rinçage final et le séchage. Certaines machines effectuent automatiquement en 30 minutes, une opération de lavage désinfection d'un seul ou de deux endoscopes en même temps selon le processus en 8 phases suivant :

| Phases | Fonctions | Durée en minutes | Température °C | Produits |
|---|---|---|---|---|
| 0 | Rinçage | 0,5 | 25 | eau filtrée |
| 1 | Lavage | 2 | 25 | Détergent alcalin |
| 2 | Rinçage | 0,5 | 25 | eau filtrée |
| 3 | Rinçage | 0,5 | 25 | eau filtrée |
| 4 | Désinfection | 10 | 25 | glutaraldéhyde à2% |
| 5 | Rinçage | 0,5 | 25 | eau filtrée |
| 6 | Rinçage | 0,5 | 25 | eau filtrée |
| 8 | Soufflage | 2 | - | air filtré |

L'étape de désinfection est mise en oeuvre avec une composition désinfectante concentrée en principes actifs, que la machine à laver et à désinfecter les endoscopes, dilue automatiquement à la concentration efficace. La température, et le temps de désinfection sont des paramètres importants de l'étape de désinfection et conditionnent sa réussite ; la température opératoire est généralement comprise entre 15° C et 25°C, mais dans certains cas peut s'élever jusqu'à 50°C.

En désinfection manuelle, l'opérateur doit injecter les agents de nettoyage puis de désinfection avec une seringue dans les canaux du dispositif en comportant, Ces étapes sont réalisées dans des bacs disposant d'un couvercle. Le désinfectant est généralement prêt à l'emploi et sa durée d'utilisation est fonction du nombre de trempages. Généralement, dans le cas des endoscopes, la solution doit être changée après une à deux semaines d'utilisation ou après 40 opérations de désinfection.

Le glutaraldéhyde à 2 % dans l'eau en désinfection manuelle, et à 20-23 % dans l'eau en désinfection automatique, est aujourd'hui la matière active la plus utilisée en Europe. Le temps de désinfection efficace avec ce produit est, en désinfection manuelle, compris entre 20 minutes dans le cas d'un risque infectieux médian, et 1 heure dans le cas d'un risque d'infection élevé. En désinfection par machines, ce temps est compris entre 5 à 20 minutes environ. Cependant, le glutaraldéhyde est toxique et il fixe les protéines. Pour cette raison il est susceptible de fixer fortement l'infectiosité résiduelle liée à la présence des ATNC. C'est pourquoi les autorités françaises recommandent son remplacement par des désinfectants qui n'ont pas cet inconvénient. De plus, le glutaraldéhyde est peu efficace contre les spores. En effet, une solution aqueuse de 2 % en poids de glutaraldéhyde est inefficace à 20 °C après 20 minutes, tant sur Bacillus cereus que sur Bacillus subtilis. Il ne devient efficace contre ces souches qu'après au moins 1 heure de trempage.

C'est pourquoi la demanderesse a recherché un substitut à ce produit. Elle s'est intéressée aux solutions aqueuses d'acide peracétique qui sont connues pour leur efficacité sur l'ensemble des microorganismes, notamment sur les spores, lorsqu'elles sont à une concentration suffisante en peracide, soit à partir d'environ 5000 ppm pour un abattement de 5 log et d'environ 2500 ppm pour un abattement de 3 log en 5 minutes.

La demande internationale publiée sous le numéro WO 01/10125 A et le brevet américain publié sous le numéro US 6,171,551 divulguent un système désinfectant à deux éléments contenant :
A - une solution aqueuse comprenant de l'acide acétique, de l'acide peracétique, du peroxyde d'hydrogène et des agents tensioactifs non-ioniques et
B - une solution aqueuse comprenant au moins deux inhibiteurs de corrosion dont au moins une des deux est un phosphate

La demanderesse a donc cherché à mettre au point un nouveau procédé de désinfection qui n'ait pas les inconvénients exposés ci-dessus.

Elle a mis au point un nouveau système désinfectant à deux éléments qui permet la préparation automatique ou manuelle d'une composition désinfectante sans danger pour l'opérateur et qui, contrairement aux systèmes désinfectants à deux éléments de l'état de la technique, comme ceux décrit dans les demandes de brevet européen publiées sous les numéros EP 609 266, EP 0 870 853 ou EP 953 283, n'engendre pas le risque d'obtenir une solution ayant une quantité insuffisance de désinfectant, du fait d'un mélange insuffisant des deux éléments du système ou d'un pH trop élevé.

Selon un premier aspect, l'invention a pour objet un procédé de préparation d'une composition désinfectante aqueuse (C), caractérisé en ce qu'il comprend :
(a) - une étape de mélange d'une solution aqueuse (A) contenant de l'acide acétique, de l'acide peracétique, du peroxyde d'hydrogène et au moins un agent tensioactif non-ionique, avec une solution aqueuse (B) contenant au moins deux inhibiteurs de corrosion dont au moins l'un des deux est un phosphate, un orthophosphate, un hydrogénophosphate ou un dihydrogénophosphate de métal alcalin et au moins un agent alcalin, dans un rapport pondéral Rₚ₁ = poids de solution (A) / poids de solution (B) supérieur ou égal à 1 / 10 et inférieur ou égal à 10/1, pour former une solution (M), et
(b) - une étape de dilution dans l'eau de la solution (M) obtenue à l'étape (a), pour former la composition (C), telle que le rapport pondéral Rₚ₂ = poids de solution (M) / poids de solution (C) soit compris entre 1 / 5 et 1 / 100, et caractérisé en ce que la composition (C) a un pH strictement inférieur à 7

Comme agents tensioactifs non ioniques présents dans la solution (A), on désigne plus particulièrement, ceux décrits dans les demandes de brevet européen publiées sous les numéros EP 0 873 687, EP 1 070 505 et EP 1 070 506, et plus particulièrement, les produits commerciaux suivants : le GENAPOL^{™} 2822, le GENAPOL^{™} 2908, le GENAPOL^{™} 2908D, le GENAPOL^{™} 2909, le TRITON^{™} DF12, le TRITON^{™} DF16, le TRITON^{™} CF10, le DOWFAX^{™} 20B102, l'AKY-PO^{™} RO 90, le SYNPERONIC^{™} LF RA 30 ou le SIMULSOL^{™} NW 900, l'AKYPO LF2 ou l'AKYPO LF4. Ces produits, disponibles dans le commerce, ont la composition chimique suivante :

| **nom commercial** | **composition chimique** |
|---|---|
| GENAPOL^{™} 2822 | Mélange d'alcools alcoxylés en C₁₀, C₁₂, C₁₄ (5OE, 4OP) |
| GENAPOL^{™} 2908 | Mélange d'alcools alcoxylés en C₁₁, C₁₃, C₁₅ (6 à 7OE, 3OP) |
| GENAPOL^{™} 2909 | Mélange d'alcools alcoxylés en C₁₂, C₁₄ (5OE, 3OP) |
| TRITON^{™} DF12 | Ethers benzyliques d'alcools alcoxylés en C₈, C₁₀ (2OE, 5OP |
| TRITON^{™} DF16 | Mélange d'alcools alcoxylés en C₈, C₁₀ (6OE, 3OP) |
| TRITON^{™} CF10 | Ether benzylique d'alcools éthoxylés en C₈ (16OE) |
| AKYPO^{™} RO 90 | Mélange d'éthers carboxyliques éthoxylés en C₁₆ C₁₈ (9 OE) |
| DOWFAX^{™}20B102 | |
| SIMULSOL^{™} NW 900 | Mélange d'alcools alcoxylés (x OE, y OP) |
| AKYPO LF2 | Ether carboxylique éthoxylé en C₈ (8 OE) |
| AKYPO LF4 | Mélange d'éthers carboxyliques éthoxylés en Ce, C₈ (7 OE) |

Comme agents tensioactifs non-ioniques, on préfère ceux correspondant à la formule (I) suivante :

R₁-O-[CH(R₂)-CH(R₃)-O]ₙ-R₄ (I)

dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle et n représente un nombre entier compris entre 1 et 50 et de préférence inférieur à 20.

Par radical hydrocarboné linéaire ou ramifié, saturé ou insaturé, comprenant de 5 à 31 atomes de carbone, on désigne pour R₁ dans la formule (I) telle que définie précédemment, notamment les radicaux alkyle ou alcènyle. R₁ représente plus particulièrement un radical choisi parmi les radicaux hexyle, heptyle, octyle, nonyle, décyle, undécyle, undécènyle, dodécyle, tridécyle, tetradécyle, pentadécyle, hexadécyle, heptadécyle, octadécyle, octadécènyle, octadécatriènyle, octadécadiènyle, eicosanyle ou béhènyle, lesdits radicaux étant linéaires ou ramifiés. R₁ représente plus particulièrement un radical choisi parmi les radicaux alkyles linéaires ou ramifiés comportant de 8 à 18 atomes de carbone.

Par radical alkyle comprenant de 1 à 4 atomes de carbone, on désigne pour R₄ dans la formule (I) telle que définie précédemment, les radicaux méthyle, éthyle, propyle ou butyle, linéaires ou ramifiés.

Le groupe divalent radical -[CH(R₂)-CH(R₃)-O]ₙ- représente, soit une chaîne composée uniquement de groupes éthoxyle (R₂ et R₃ = H), soit une chaîne composée uniquement de groupes propoxyle (R₂ ou R₃ = CH₃), soit une chaîne composée uniquement de groupes butoxyle (R₂ et R₃ = CH₃) soit une chaîne composée d'un mélange de deux sortes ou des trois sortes de groupes énoncés ci-dessus. Dans ce dernier cas, les fragments -CH₂-CH₂-O- et / ou -CH(CH₃)-CH₂-O- et / ou -CH(CH₃)-CH(CH₃)-O-, sont distribués dans ladite chaîne, de façon séquencée ou aléatoire.

Comme inhibiteurs de corrosion présents dans la solution (B) avec les sels de l'acide phosphorique ou des dérivés dudit acide, on désigne plus particulièrement les dérivés du triazole ou de l'imidazole comme par exemple, le benzotriazole, le tolyl triazole ou le benzimidazole.

Comme agent alcalin présent dans la solution (B), on désigne essentiellement l'hydroxyde de potassium ou l'hydroxyde de sodium.

Selon un premier aspect particulier de la présente invention, la solution aqueuse (A) comporte entre 2 % et 20 % en poids d'acide peracétique, généralement entre 5 % et 16 % en poids.

Selon un deuxième aspect particulier de la présente invention, la solution (A) est une solution aqueuse à l'équilibre comportant entre 2 % et 20 % en poids d'acide peracétique, généralement entre 5 % et 16 % en poids.

Selon un troisième aspect particulier de la présente invention, la solution aqueuse (A) comporte entre 5 % et 40 % en poids de peroxyde d'hydrogène, généralement entre 10 % et 30 % en poids.

Selon un quatrième aspect particulier de la présente invention, la solution aqueuse (A) comporte entre 5 % et 30 % en poids d'acide acétique, généralement ente 10 % et 25 % en poids.

Selon un cinquième aspect particulier de la présente invention, la solution aqueuse (A) comporte, entre 0,01 % et 1% en poids d'agent tensioactif non ionique.

Selon un sixième aspect particulier de la présente invention, la solution aqueuse (B) comporte entre 0,1 % et 5 % en poids de benzotriazole.

Selon un septième aspect particulier de la présente invention, la solution aqueuse (B) comporte entre 0,5 % à 15 % en poids d'au moins un sel alcalin de l'acide phosphorique ou des dérivés dudit acide, généralement entre 5 % et 15 % en poids.

Selon un huitième aspect particulier de la présente invention, la solution aqueuse (B) comporte 2 % et 30 % en poids de d'au moins un hydroxyde alcalin, généralement entre 5 % et 20 % en poids.

Selon un neuvième aspect particulier de la présente invention, celle-ci a pour objet l'invention a aussi pour objet un procédé de préparation de la composition (C) tel que défini précédemment, dans lequel le rapport pondéral Rₚ₁ est supérieur ou égal à 1 / 5 et inférieur ou égal à 5 / 1 et est de préférence compris entre 1 / 2 et 2 / 1.

Selon un dixième aspect particulier de la présente invention, la composition (C) a un pH inférieur à 6,5 et tout particulièrement un pH inférieur à 6.

Selon un autre aspect particulier de la présente invention, ladite composition (C), contient généralement entre 0,01 % et 4 % en poids de peroxyde d'hydrogène et selon encore un autre aspect particulier de la présente invention, ladite composition (C), contient entre 0,01 % et 4 % en poids d'acide acétique.

L'invention a plus particulièrement pour objet un procédé tel que défini précédemment, dans lequel la composition (C) mise en contact avec le dispositif à désinfecter, comporte entre 0,001% et 0,25 % en poids de benzotriazole, entre 0,005 % et 0,5 % en poids d'orthophosphate de sodium.

Un dispositif médical est désinfecté en le mettant en contact avec la composition (C) obtenu par le procédé tel que défini précédemment.

Le procédé de désinfection tel que défini ci-dessus, peut être un procédé manuel, automatique ou semi-automatique.

Par dispositif médical, on désigne de façon générale, tout dispositif non autoclavable et plus particulièrement :
- les instrumentations et moteurs de microchirurgie et coelio - chirurgie,
- les pièces à mains en dentisterie, en stomatologie et en chirurgie,
- le matériel biofeedback en urologie,
- les dispositifs médicaux en ophtalmologie, tels que par exemple, les prismes à trois miroirs ou les tonomètres à aplanation,
- et de nombreux endoscopes tels que par exemple les bronchoscopes, les laryngoscopes, les oesophagoscopes, les gastroscopes, les colonoscopes, les rectoscopes, les laparoscopes, les arthroscopes, les cystoscopes, les amnioscopes, les médiastinoscopes, les hystéroscopes, les coelioscopes, les sinuscopes, les cholédoscopes transpariétaux, les cholesdoscopes rétrogrades ou les urétéroscopes.

La mise en contact du dispositif avec la composition (C) telle que définie précédemment, se fait par aspersion et à l'intérieur des canaux, par injection de la solution au moyen d'une pompe et/ou par trempage.

Le temps de contact avec la composition est en général inférieur à 20 minutes et il est plus particulièrement compris entre 5 et 15 minutes.

Selon la réglementation en vigueur dans le pays de leur utilisation et le cas échéant selon la machine à laver utilisée, le procédé est mis en oeuvre à une température comprise entre 15°C et 40°C environ.

Dans le procédé désinfection tel que défini précédemment, l'étape de mise en contact dudit matériel avec ladite solution est précédée d'au moins une étape de nettoyage.

Par étape de nettoyage, on indique une étape dont l'objectif est d'éliminer les salissures et qui va conjuguer l'action physico-chimique de la solution détergente utilisée dans cette étape utilisée et des actions mécaniques effectuées par la machine, telles que le pompage de la solution de lavage dans les canaux de l'endoscope ou l'aspersion.

Dans le procédé désinfection tel que défini précédemment, l'étape de nettoyage dudit matériel, est précédée d'une étape d'une étape de pré-traitement.

Par étape de pré-traitement, on indique une étape dont le but consiste à faciliter les étapes ultérieures, en empêchant notamment les salissures de sécher, par exemple en irriguant immédiatement après la fin de leur utilisation, le canal de l'instrument médical en comportant, par une solution détergente et éventuellement bactéricide et/ou en l'immergeant dans ladite solution.

Lorsque le procédé de désinfection est effectué en machine, cette étape est effectuée à l'extérieur de la machine.

Dans le procédé désinfection tel que défini précédemment, l'étape de mise en contact dudit matériel avec ladite solution est suivie d'une étape de rinçage puis, si nécessaire, d'une étape de séchage.

L'étape de rinçage final a pour objectif est d'éliminer tout résidu de produit tout en évitant les re-contaminations ; ceci implique l'utilisation d'une eau adaptée à cette exigence qui, selon le niveau de risque, sera stérile ou filtrée ; le séchage est nécessaire lorsque le dispositif n'est pas immédiatement réutilisé.

Les exemples suivants illustrent l'invention sans toutefois la limiter.

### A) détermination de l'effet protecteur contre la corrosion des compositions selon l'invention.

### Exemple 1

### (i) - On procède à des essais de corrosion de plaques métalliques en cuivre et en aluminium ayant les caractéristiques suivantes :

### Plaques d'aluminium

teneur en Al : 99% ; épaisseur : 0,025 cm ; largeur : 2,5 cm ; longueur : 5,0 cm.

### Plaques de cuivre

teneur en Cu : 99% ; épaisseur : 0,030 cm ; largeur : 2,5 cm ; longueur : 5,0 cm.

Chaque plaque testée subit 16 trempages successifs de 5 minutes, dans un bécher de 150 ml contenant 100 ml d'une composition Cᵢ ou d'une composition témoins Tᵢ, (soit une profondeur d'immersion d'environ 4,5 cm).

### (ii) - Les compositions Cᵢ et les compositions témoins Tᵢ, sont préparées à partir des compositions Aᵢ et Bᵢ suivantes (pourcentages pondéraux) :

| | A₁ | A₂ | B₁ | B₂ | B₃ | B₄ |
|---|---|---|---|---|---|---|
| Acide peracétique | 10 % | 5,5 % | 0 | 0 | 0 | 0 |
| Peroxyde d'hydrogène | 18 % | 14,5 % | 0 | 0 | 0 | 0 |
| Acide acétique | 16 % | 16,5 % | 0 | 0 | 0 | 0 |
| Génapol^{™} 2908 | 0,1 % | 0,24 % | 0 | 0 | 0 | 0 |
| Benzotriazole | 0 | 0 | 1,66 % | 3,33 % | 6,7 % | 0 |
| Na₂HPO₄, 12 H₂O | 0 | 0 | 6 % | 12 % | 12 % | 0 |
| KOH | 0 | 0 | 0 | 17% | 16,5% | 18% |

par mélange et dilution dans l'eau dans les proportions suivantes (volumes exprimés en ml) :

| | A₁ | A₂ | B₁ | B₂ | B₃ | B₄ | H₂O |
|---|---|---|---|---|---|---|---|
| T₁ | 150 | 0 | 0 | 0 | 0 | 0 | 9850 |
| T₂ | 150 | 0 | 0 | 0 | 0 | 150 | 9700 |
| C₁ | 150 | 0 | 300 | 0 | 0 | 0 | 9550 |
| C₂ | 150 | 0 | 0 | 150 | 0 | 0 | 9700 |
| C₃ | 150 | 0 | 0 | 0 | 150 | 0 | 9700 |
| C₄ | 0 | 240 | 480 | 0 | 0 | 0 | 9280 |

### (iii) - Les résultats sont exprimés en pourcentage de perte de poids des pièces métalliques ainsi qu'en l'aspect de la plaque à l'issue du traitement (non corrodée (n.c.) légèrement corrodée (piqué) ; corrodée (c.)).

| | | | | |
|---|---|---|---|---|
| | pH de la composition | Température de la composition | Pièces en Al aspect | Pièces en Cu aspect |
| T₁ | 3,15 | 37 °C | -0,11 %; c. | -3,5 % ; c. |
| T₂ | 7,2 | 37 °C | 0% ; n.c. | -0,12 % ; c. |
| C₁ | 3,7 | 37 °C | 0% ; n.c. | 0% ; n.c. |
| C₂ | 6,85 | 37 °C | 0% ; n.c. | 0% ; n.c. |
| C₃ | 6,85 | 37 °C | 0% ; n.c. | 0% ; n.c. |
| C₄ | 3,7 | 20 °C | 0% ; n.c. | 0% ; n.c. |

Cet essai démontre l'intérêt de la présence d'inhibiteurs de corrosion, dans la composition selon l'invention.

### Exemple 2

### (i) - On procède à des essais de corrosion de plaques métalliques en cuivre et en aluminium ayant les caractéristiques suivantes :

### Plaques d'aluminium

teneur en Al : 99% ; épaisseur : 0,025 cm ; largeur : 2,5 cm ; longueur : 5,0 cm. Plaques de cuivre
teneur en Cu : 99% ; épaisseur : 0,030 cm ; largeur : 2,5 cm ; longueur : 5,0 cm.

Chaque plaque testée subit 16 trempages successifs de 10 minutes, dans un bécher de 150 ml contenant 100 ml d'une composition Cᵢ ou d'une composition témoins Tᵢ, (soit une profondeur d'immersion d'environ 4,5 cm).

### (ii) - Les compositions Cᵢ et les compositions témoins Tᵢ, sont préparées à partir des compositions Aᵢ et Bᵢ suivantes (pourcentages pondéraux) :

| | A₁ | A₂ | B₁ | B₂ | B₃ | B₅ |
|---|---|---|---|---|---|---|
| Acide peracétique | 10 % | 5,5 % | 0 | 0 | 0 | 0 |
| Peroxyde d'hydrogène | 18 % | 14,5 % | 0 | 0 | 0 | 0 |
| Acide acétique | 16 % | 16,5 % | 0 | 0 | 0 | 0 |
| Génapol^{™} 2908 | 0,1 % | 0,24 % | 0 | 0 | 0 | 0 |
| Benzotriazole | 0 | 0 | 1,66 % | 3,33 % | 6,7 % | 0 |
| Na₂HPO₄, 12 H₂O | 0 | 0 | 6 % | 12 % | 12 % | 0 |
| KOH | 0 | 0 | 0 | 17% | 16,5% | 16% |

par mélange et dilution dans l'eau dans les proportions suivantes (volumes exprimés en ml) :

| | A₁ | A₂ | B₁ | B₂ | B₃ | B₅ | H₂O |
|---|---|---|---|---|---|---|---|
| T₃ | 0 | 240 | 0 | 0 | 0 | 0 | 9760 |
| T₄ | 0 | 240 | 0 | 0 | 0 | 240 | 9520 |
| C₁ | 150 | 0 | 300 | 0 | 0 | 0 | 9550 |
| C₂ | 150 | 0 | 0 | 150 | 0 | 0 | 9700 |
| C₃ | 150 | 0 | 0 | 0 | 150 | 0 | 9700 |
| C₄ | | 240 | 480 | 0 | 0 | 0 | 9280 |
| C₅ | 0 | 240 | 960 | 0 | 0 | 0 | 8800 |

Les résultats sont exprimés en pourcentage de perte de poids des pièces métalliques ainsi qu'en l'aspect de la plaque à l'issue du traitement.

| | Pièces en Cu aspect | température de la composition | pH de la composition |
|---|---|---|---|
| T₃ | - 4,2 % ; c. | 20 °C | 3,05 |
| T₄ | - 0,37 % ; c | 20 °C | 7,2 |
| C₁ | 0 % ; (piqué) | 37 °C | 3,7 |
| C₂ | 0 %; n.c. | 37 °C | 6,85 |
| C₃ | 0 % ; n.c. | 37 °C | 6,85 |
| C₄ | 0 % ; (piqué) | 20 °C | 3,7 |
| C₅ | 0 % ; n.c. | 20 °C | 4,05 |

### Exemple 3

### (i) - On procède à des essais de corrosion de plaques métalliques en cuivre et en aluminium ayant les caractéristiques suivantes :

### Plaques d'aluminium

teneur en Al : 99% ; épaisseur : 0,025 cm ; largeur : 2,5 cm ; longueur : 5,0 cm.

### Plaques de cuivre

teneur en Cu : 99% ; épaisseur : 0,030 cm ; largeur : 2,5 cm ; longueur : 5,0 cm.

Chaque plaque testée subit 2 trempages successifs de 150 minutes, dans un bécher de 150 ml contenant 100 ml d'une composition Cᵢ ou d'une composition témoins Tᵢ, (soit une profondeur d'immersion d'environ 4,5 cm).

### (ii) - Les compositions Cᵢ et les compositions témoins Tᵢ, sont préparées à partir des compositions Aᵢ et Bᵢ suivantes (pourcentages pondéraux) :

| | A₁ | A₂ | B₁ | B₂ | B₃ | B₆ |
|---|---|---|---|---|---|---|
| Acide peracétique | 10 % | 5,5 % | 0 | 0 | 0 | 0 |
| Peroxyde d'hydrogène | 18 % | 14,5 % | 0 | 0 | 0 | 0 |
| Acide acétique | 16 % | 16,5 % | 0 | 0 | 0 | 0 |
| Génapol^{™} 2908 | 0,1 % | 0,24 % | 0 | 0 | 0 | 0 |
| Benzotriazole | 0 | 0 | 1,66 % | 3,33 % | 6,7 % | 3,33 % |
| Na₂HPO₄,12 H₂O | 0 | 0 | 6 % | 12 % | 12 % | 12 % |
| KOH | 0 | 0 | 0 | 17 % | 16,5 % | 13,5 % |

Les compositions Ci et Ti sont préparées comme indiqué dans le tableau suivant (volumes exprimés en ml):

| | A₁ | A₂ | B₁ | B₂ | B₃ | B₆ | H₂O |
|---|---|---|---|---|---|---|---|
| C₁ | 150 | 0 | 300 | 0 | 0 | 0 | 9550 |
| C₂ | 150 | 0 | 0 | 150 | 0 | 0 | 9700 |
| C₃ | 150 | 0 | 0 | 0 | 150 | 0 | 9700 |
| C₄ | 0 | 240 | 480 | 0 | 0 | 0 | 9280 |
| C₆ | 150 | 0 | 0 | 0 | 0 | 150 | 9700 |

Les résultats sont exprimés en pourcentage de perte de poids des pièces métalliques ainsi qu'en l'aspect de la plaque à l'issue du traitement.

| | Pièces en cuivre aspect : | température de la composition | pH de la composition |
|---|---|---|---|
| C1 | -0,1 % ; c.(piqures) | 37 °C | 3,7 |
| C2 | 0 % ; n.c. | 37 °C | 6,85 |
| C3 | 0 % ; n.c. | 37 °C | 6,85 |
| C4 | 0 %; c. (piqures) | 20 °C | 3,7 |
| C6 | 0 % ; n.c. | 37 °C | 5,65 |

Cet essai démontre l'avantage combiné d'être à acide et en présence de deux inhibiteurs de corrosion dans la solution pour des trempages de longues périodes.

### B-Activité sporicide des compositions selon l'invention.

Les activités sporicides des compositions C₁, C₂ et C₆ préparées comme au paragraphe A, a été évaluée sur Bacillus Cereus CIP 78-3, selon la norme NFT 72-301.

Les résultats sont exprimés en abattement logarithmique de la population de microorganismes.

| | temps de contact :5 min. T=37°C | temps de contact :10 min. T=20°C |
|---|---|---|
| C₁, | 4 log. | 4 log. |
| C₂, | 2 log. | 2 log. |
| C₆ | 3 log | 3 log. |

Les résultats font apparaître que l'objectif d'abattement établi à 3 log est atteint aisément par les compositions selon l'invention.

## Revendications

1. Procédé de préparation d'une composition désinfectante aqueuse (C), **caractérisé en ce qu'**il comprend :
(a) - une étape de mélange d'une solution aqueuse (A) contenant de l'acide acétique, de l'acide peracétique, du peroxyde d'hydrogène et au moins un agent tensioactif non-ionique, avec une solution aqueuse (B) contenant au moins deux inhibiteurs de corrosion dont au moins l'un des deux est un phosphate, un orthophosphate, un hydrogénophosphate ou un dihydrogénophosphate de métal alcalin et au moins un agent alcalin, dans un rapport pondéral Rₚ₁ = poids de solution (A) / poids de solution (B) supérieur ou égal à 1 / 10 et inférieur ou égal à 10 / 1, pour former une solution (M), et
(b) - une étape de dilution dans l'eau de la solution (M) obtenue à l'étape (a), pour former la composition (C), telle que le rapport pondéral Rₚ₂ = poids de solution (M) / poids de solution (C) soit compris entre 1 / 5 et 1 / 100, et **caractérisé en ce que** la composition (C) a un pH strictement inférieur à 7.

2. Procédé tel que défini à la revendication 1, pour lequel dans la solution (A), les agents tensioactifs non ioniques sont des composés de formule (I) :
R₁-O-[CH(R₂)-CH(R₃)-O]ₙ-R₄ (I)
dans laquelle R₁ représente un radical hydrocarboné linéaire ou ramifié, saturé ou insaturé comprenant de 5 à 31 atomes de carbones, R₂ et R₃ représentent indépendamment l'un de l'autre, un atome d'hydrogène ou un radical alkyle comprenant 1 ou 2 atomes de carbone, R₄ représente un atome d'hydrogène, un radical alkyle linéaire ou ramifié comprenant de 1 à 4 atomes de carbone ou un radical benzyle, et n représente un nombre entier compris entre 1 et 50 et de préférence inférieur à 20.

3. Procédé tel que défini à la revendication 2, pour lequel dans la formule (I), R₁ représente un radical choisi parmi les radicaux alkyles linéaires ou ramifiés comportant de 8 à 18 atomes de carbone.

4. Procédé tel que défini à l'une des revendications 1 à 3, pour lequel la solution aqueuse (A) comporte entre 2 % et 20 % en poids d'acide peracétique.

5. Procédé tel que défini à l'une des revendications 1 à 4, pour lequel la solution (A) est une solution aqueuse à l'équilibre comportant entre 2 % et 20 % en poids d'acide peracétique.

6. Procédé tel que défini à l'une des revendications 1 à 5, pour lequel la solution aqueuse (A) comporte entre 5 % et 40 % en poids de peroxyde d'hydrogène.

7. Procédé tel que défini à l'une des revendications 1 à 6, pour lequel la solution aqueuse (A) comporte entre 10 % et 25 % en poids d'acide acétique.

8. Procédé tel que défini à l'une des revendications 1 à 7, pour lequel la solution aqueuse (A) comporte, entre 0,01 % et 1% en poids d'agent tensioactif non ionique.

9. Procédé tel que défini à l'une des revendications 1 à 8, pour lequel la solution aqueuse (B) comporte entre 0,1 % et 5 % en poids de benzotriazole.

10. Procédé tel que défini à l'une des revendications 1 à 9, pour lequel la solution aqueuse (B) comporte entre 0,5 % à 15 % en poids d'au moins un sel alcalin de l'acide phosphorique ou des dérivés dudit acide.

11. Procédé tel que défini à l'une des revendications 1 à 10, pour lequel la solution aqueuse (B) comporte 2 % et 30 % en poids d'au moins un hydroxyde alcalin.

12. Procédé tel que défini à l'une quelconque des revendications 1 à 11, dans lequel la composition (C) a un pH inférieur à 6,5.

13. Procédé tel que défini à la revendication 12, dans lequel la composition (C) a un pH inférieur à 6.

14. Procédé tel que défini à l'une quelconque des revendications 1 à 13, dans lequel la composition (C) a un pH supérieur à 3,5.

15. Procédé tel que défini à la revendication 14 dans lequel la composition (C) a un pH supérieur à 4.

16. Procédé de préparation de la composition (C) tel que défini à l'une quelconque des revendications 1 à 15, dans lequel le rapport pondéral Rₚ₁ est supérieur ou égal à 1 / 5 et inférieur ou égal à 5 /1.

17. Procédé de préparation de la composition (C) tel que défini à la revendication 16, dans lequel le rapport pondéral Rₚ₁ est compris entre 1 / 2 et 2 / 1.

18. Procédé de préparation de la composition (C) tel que défini à l'une quelconque des revendications 1 à 17, **caractérisé en ce que** ladite composition (C), contient entre environ 0,01 % et environ 1,0 % en poids d'acide peracétique.

19. Procédé de préparation de la composition (C) tel que défini à la revendication 18, **caractérisé en ce que** ladite composition (C), contient entre environ 0,08 % et environ 0,35 % en poids d'acide peracétique, et plus particulièrement entre 0,09 % et 0,20 % en poids d'acide peracétique.

20. Procédé de préparation de la composition (C) tel que défini à l'une quelconque des revendications 1 à 19, **caractérisé en ce que** ladite composition (C), contient entre 0,01 % et 4 % en poids de peroxyde d'hydrogène.

21. Procédé de préparation de la composition (C) tel que défini à l'une quelconque des revendications 1 à 20, **caractérisé en ce que** ladite composition (C), contient entre 0,01 % et 4 % en poids d'acide acétique.

22. Procédé de préparation de la composition (C) tel que défini à l'une quelconque des revendications 1 à 21, **caractérisé en ce que** ladite composition (C), contient entre 0,001% et 0,25 % en poids de benzotriazole, entre 0,005 % et 0,5 % en poids d'orthophosphate de sodium.

## Claims

1. Process for the preparation of an aqueous disinfecting composition (C), **characterized in that** it comprises:
(a) - a stage of mixing an aqueous solution (A) comprising acetic acid, peracetic acid, hydrogen peroxide and at least one non-ionic surface-active agent with an aqueous solution (B) comprising at least two corrosion inhibitors, at least one of the two of which is an alkali metal phosphate, orthophosphate, hydrogenphosphate or dihydrogenphosphate, and at least one alkaline agent, in a ratio by weight R_{w1} = weight of solution (A)/ weight of solution (B) of greater than or equal to 1/10 and less than or equal to 10/1, to form a solution (M), and
(b) - a stage of diluting, in water, the solution (M) obtained in stage (a), to form the composition (C), such that the ratio by weight R_{w2} = weight of solution (M)/weight of solution (C) is between 1/5 and 1/100,
and **characterized in that** the composition (C) has a pH strictly of less than 7.

2. Process as defined in Claim 1, for which, in the solution (A), the non-ionic surface-active agents are compounds of formula (I):
R₁-O- [CH(R₂) -CH(R₃) -O]ₙ-R₄ (I)
in which R₁ represents a saturated or unsaturated and linear or branched hydrocarbon radical comprising from 5 to 31 carbon atoms, R₂ and R₃ represent, independently of one another, a hydrogen atom or an alkyl radical comprising 1 or 2 carbon atoms, R₄ represents a hydrogen atom, a linear or branched alkyl radical comprising from 1 to 4 carbon atoms or a benzyl radical, and n represents an integer between 1 and 50 and preferably of less than 20.

3. Process as defined in Claim 2, for which, in the formula (I), R₁ represents a radical chosen from linear or branched alkyl radicals comprising from 8 to 18 carbon atoms.

4. Process as defined in one of Claims 1 to 3, for which the aqueous solution (A) comprises between 2% and 20% by weight of peracetic acid.

5. Process as defined in one of Claims 1 to 4, for which the solution (A) is a balanced aqueous solution comprising between 2% and 20% by weight of peracetic acid.

6. Process as defined in one of Claims 1 to 5, for which the aqueous solution (A) comprises between 5% and 40% by weight of hydrogen peroxide.

7. Process as defined in one of Claims 1 to 6, for which the aqueous solution (A) comprises between 10% and 25% by weight of acetic acid.

8. Process as defined in one of Claims 1 to 7, for which the aqueous solution (A) comprises between 0.01% and 1% by weight of non-ionic surface-active agent.

9. Process as defined in one of Claims 1 to 8, for which the aqueous solution (B) comprises between 0.1% and 5% by weight of benzotriazole.

10. Process as defined in one of Claims 1 to 9, for which the aqueous solution (B) comprises between 0.5% and 15% by weight of at least one alkaline salt of phosphoric acid or the derivatives of the said acid.

11. Process as defined in one of Claims 1 to 10, for which the aqueous solution (B) comprises between 2% and 30% by weight of at least one alkaline hydroxide.

12. Process as defined in any one of Claims 1 to 11, in which the composition (C) has a pH of less than 6.5.

13. Process as defined in Claim 12, in which the composition (C) has a pH of less than 6.

14. Process as defined in any one of Claims 1 to 13, in which the composition (C) has a pH of greater than 3.5.

15. Process as defined in Claim 14, in which the composition (C) has a pH of greater than 4.

16. Process for the preparation of the composition (C) as defined in any one of Claims 1 to 15, in which the ratio by weight R_{w1} is greater than or equal to 1/5 and less than or equal to 5/1.

17. Process for the preparation of the composition (C) as defined in Claim 16, in which the ratio by weight R_{w1} is between 1/2 and 2/1.

18. Process for the preparation of the composition (C) as defined in any one of Claims 1 to 17, **characterized in that** the said composition (C) comprises between approximately 0.01% and approximately 1.0% by weight of peracetic acid.

19. Process for the preparation of the composition (C) as defined in Claim 18, **characterized in that** the said composition (C) comprises between approximately 0.08% and approximately 0.35% by weight of peracetic acid and more particularly between 0.09% and 0.20% by weight of peracetic acid.

20. Process for the preparation of the composition (C) as defined in any one of Claims 1 to 19, **characterized in that** the said composition (C) comprises between 0.01% and 4% by weight of hydrogen peroxide.

21. Process for the preparation of the composition (C) as defined in any one of Claims 1 to 20, **characterized in that** the said composition (C) comprises between 0.01% and 4% by weight of acetic acid.

22. Process for the preparation of the composition (C) as defined in any one of Claims 1 to 21, **characterized in that** the said composition (C) comprises between 0.001% and 0.25% by weight of benzotriazole and between 0.005% and 0.5% by weight of sodium orthophosphate.

## Patentansprüche

1. Verfahren zur Herstellung einer wässrigen Desinfektionszusammensetzung (C), **dadurch gekennzeichnet, dass** es :
(a) einen Schritt des Mischens einer wässrigen Lösung (A), die Essigsäure, Peressigsäure, Wasserstoffperoxid und mindestens ein nichtionisches oberflächenaktives Agens enthält, mit einer wässrigen Lösung (B), die mindestens zwei Korrosionsinhibitoren enthält, wobei mindestens einer der beiden ein Phosphat, ein Orthophosphat, ein Hydrogenphosphat oder ein Dihydrogenphosphat eines Alkalimetalls ist sowie mindestens ein alkalisches Agens in einem Gewichtsverhältnis Rₚ₁ = Gewicht der Lösung (A)/ Gewicht der Lösung (B), das größer oder gleich 1/10 und kleiner oder gleich 10/1 ist, zur Bildung einer Lösung (M) sowie
(b) einen Schritt der Verdünnung der in Schritt (a) erhaltenen Lösung (M) in Wasser zur Bildung einer Zusammensetzung (C), so dass das Gewichtsverhältnis Rₚ₂ = Gewicht der Lösung (M)/ Gewicht der Lösung (C) zwischen 1/5 und 1/100 liegt, umfasst
und **dadurch gekennzeichnet, dass** die Lösung (C) einen pH aufweist, der strikt unterhalb von 7 liegt.

2. Verfahren nach Anspruch 1, wobei in der Lösung (A) die nicht-ionischen oberflächenaktiven Agentien Verbindungen der Formel (I) sind:
R₁-O-[CH(R₂)-CH(R₃)-O]ₙ-R₄ (I)
in der R₁ ein lineares oder verzweigtes, gesättigtes oder ungesättigtes Kohlenwasserstoffradikal darstellt, das 5 bis 31 Kohlenstoffatome umfasst, R₂ und R₃ unabhängig voneinander ein Wasserstoffatom oder ein Alkylradikal darstellen, das 1 oder 2 Kohlenstoffatome umfasst, R₄ ein Wasserstoffatom, ein lineares oder verzweigtes Alkylradikal, das 1 bis 4 Kohlenstoffatome umfasst oder ein Benzylradikal darstellt und n eine ganze Zahl zwischen 1 und 50 und vorzugsweise unter 20 darstellt.

3. Verfahren nach Anspruch 2, wobei in der Formel (I) R₁ ein Radikal darstellt, das ausgewählt ist unter linearen oder verzweigten Alkylradikalen, die 8 bis 18 Kohlenstoffatome umfassen.

4. Verfahren nach einem der Ansprüche 1 bis 3, wobei die Lösung (A) 2 Gew.% bis 20 Gew.% Peressigsäure umfasst .

5. Verfahren nach einem der Ansprüche 1 bis 4, wobei die Lösung (A) eine wässrige Lösung im Gleichgewicht ist, die zwischen 2 Gew.% und 20 Gew.% Peressigsäure umfasst.

6. Verfahren nach einem der Ansprüche 1 bis 5, wobei die wässrige Lösung (A) zwischen 5 Gew.% und 40 Gew.% Wasserstoffperoxid umfasst.

7. Verfahren nach einem der Ansprüche 1 bis 6, wobei die wässrige Lösung (A) zwischen 10 Gew.% und 25 Gew.% Essigsäure umfasst.

8. Verfahren nach einem der Ansprüche 1 bis 7, wobei die wässrige Lösung (A) zwischen 0,01 Gew.% und 1 Gew.% eines nicht-ionischen oberflächenaktiven Agens umfasst.

9. Verfahren nach einem der Ansprüche 1 bis 8, wobei die wässrige Lösung (B) zwischen 0,1 Gew.% und 5 Gew.% Benzotriazol umfasst.

10. Verfahren nach einem der Ansprüche 1 bis 9, wobei die wässrige Lösung (B) zwischen 0,5 Gew.% und 15 Gew.% mindestens eines alkalischen Salzes von Phosphorsäure oder Derivaten dieser Säure umfasst.

11. Verfahren nach einem der Ansprüche 1 bis 10, wobei die wässrige Lösung (B) zwischen 2 Gew.% und 30 Gew.% mindestens eines alkalischen Hydroxids umfasst.

12. Verfahren nach einem der Ansprüche 1 bis 11, wobei die Zusammensetzung (C) einen pH unterhalb von 6,5 aufweist.

13. Verfahren nach Anspruch 12, wobei die Zusammensetzung (C) einen pH unterhalb von 6 aufweist.

14. Verfahren nach einem der Ansprüche 1 bis 13, wobei die Zusammensetzung (C) einen pH oberhalb von 3,5 aufweist.

15. Verfahren nach Anspruch 14, wobei die Zusammensetzung (C) einen pH oberhalb von 4 aufweist.

16. Verfahren zur Herstellung der Zusammensetzung (C) nach einem der Ansprüche 1 bis 15, wobei das Gewichtsverhältnis Rₚ₁ größer oder gleich 1/5 und kleiner oder gleich 5/1 ist.

17. Verfahren zur Herstellung der Zusammensetzung (C) nach Anspruch 16, wobei das Gewichtsverhältnis Rp₁ zwischen 1/2 und 2/1 liegt.

18. Verfahren zur Herstellung der Zusammensetzung (C) nach einem der Ansprüche 1 bis 17, **dadurch gekennzeichnet, dass** die Zusammensetzung (C) zwischen etwa 0,01 Gew.% und etwa 1,0 Gew.% Peressigsäure enthält.

19. Verfahren zur Herstellung der Zusammensetzung (C) nach Anspruch 18, **dadurch gekennzeichnet, dass** die Zusammensetzung (C) zwischen etwa 0,08 Gew.% und etwa 0,35 Gew.% Peressigsäure enthält und besonders bevorzugt zwischen 0,09 Gew.% und 0,20 Gew.% Peressigsäure.

20. Verfahren zur Herstellung der Zusammensetzung (C) nach einem der Ansprüche 1 bis 19, **dadurch gekennzeichnet, dass** die Zusammensetzung (C) zwischen 0,01 Gew.% und 4 Gew.% Wasserstoffperoxid enthält.

21. Verfahren zur Herstellung der Zusammensetzung (C) nach einem der Ansprüche 1 bis 20, **dadurch gekennzeichnet, dass** die Zusammensetzung (C) zwischen 0,01 Gew.% und 4 Gew.% Essigsäure enthält.

22. Verfahren zur Herstellung der Zusammensetzung (C) nach einem der Ansprüche 1 bis 21, **dadurch gekennzeichnet, dass** die Zusammensetzung (C) zwischen 0,001 Gew.% und etwa 0,25 Gew.% Benzotriazol und zwischen 0,005 Gew.% und 0,5 Gew.% Natriumorthophosphat enthält.
